(19) Europäisches Patentamt
European Patent Office
Office européen des brevets

(11) EP 1 110 950 B1

(12) EUROPÄISCHE PATENTSCHRIFT

(45) Veröffentlichungstag und Bekanntmachung des Hinweises auf die Patenterteilung:
**09.07.2003 Patentblatt 2003/28**

(51) Int Cl.[7]: **C07D 209/52**

(21) Anmeldenummer: **00121160.6**

(22) Anmeldetag: **29.09.2000**

# (54) Chromatographische Enantiomerentrennung von bicyclischen Lactamen

Chromatographic separation of enantiomers of bicyclic lactames

Séparation chromatographique d'énantiomères de lactames bicycliques

(84) Benannte Vertragsstaaten:
**AT BE CH CY DE DK ES FI FR GB GR IE IT LI LU MC NL PT SE**

(30) Priorität: **23.12.1999 DE 19962543**

(43) Veröffentlichungstag der Anmeldung:
**27.06.2001 Patentblatt 2001/26**

(73) Patentinhaber: **Degussa AG**
**40474 Düsseldorf (DE)**

(72) Erfinder:
- **Günther, Kurt, Dr.**
  **63526 Erlensee (DE)**
- **Merget, Stefan**
  **63110 Rodgau (DE)**
- **Drauz, Karlheinz, Prof.**
  **63579 Freigericht (DE)**
- **Krimmer, Hans-Peter, Dr.**
  **63128 Dietzenbach (DE)**

(56) Entgegenhaltungen:
**EP-A- 0 424 064**
**EP-A- 0 816 339**
**WO-A-98/24741**
**WO-A-99/10519**
**DE-A- 19 958 497**
**DE-A- 19 958 498**
**DE-C- 19 707 641**

Bemerkungen:
Die Akte enthält technische Angaben, die nach dem Eingang der Anmeldung eingereicht wurden und die nicht in dieser Patentschrift enthalten sind.



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung ist auf ein Verfahren zur Auftrennung der Enantiomere des Lactams (I)

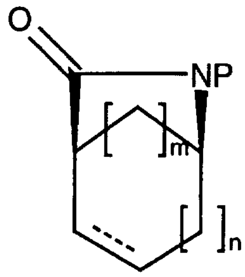

(I)

gerichtet.

Diese sind Ausgangsmaterialien zur Herstellung von carbozyclischen Nukleosidanalogen, die wegen ihrer antiviralen und chemotherapeutischen Eigenschaften als Heilmittel in der Medizin von Interesse sind.

Bisher werden derartige Verbindungen bevorzugt durch enzymatische Racematspaltung aus den entsprechenden Racematen hergestellt (WO99/10519).

Die EP 424 064 beschreibt die enzymatische Lactamöffnung von speziellen 2-Azabicyclo[2.2.1]heptenonen und verwandten Derivaten.

In der DE 197 07 641 wird über die chromatographische Enantiomerentrennung von 5-Heteroaryl-1,3,4-thiadiazinonen berichtet.

Der Nachteil der enzymatischen Enantiomerentrennung für einen großtechnischen Prozeß ist die Notwendigkeit der Produktion im Batchbetrieb. Außerdem ist die Produktion und das Handling der benutzten Enzyme aufwendig und kostspielig, so daß der Gesamtprozeß ökonomisch gesehen unvorteilhaft erscheint.

Aufgabe der vorliegenden Erfindung ist deshalb die Bereitstellung eines weiteren Verfahrens zur Enantiomerentrennung von derartigen bicyclischen Lactamen. Insbesondere sollte das Verfahren mit weniger hohen Produktionskosten auskommen.

Dadurch, daß man zur Enantiomerentrennung von Lactamen der allgemeinen Formel (I)

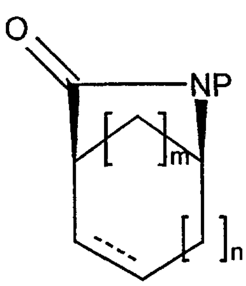

(I)

worin n, m unabhängig voneinander die Werte 0 bis 3 annehmen können,

P = H oder eine N-Schutzgruppe bedeutet,

die Methode der Flüssigkeitschromatographie an Zuckerderivate aufweisenden chiralen Phasen mittels eines Eluenten, der Acetonitril aufweist, anwendet, gelangt man in überraschend einfacher aber damit nicht weniger vorteilhafter Weise zu entsprechend hohen Trennfaktoren für die betrachteten Gemische, die ursächlich für den ökonomisch vorteilhaften Einsatz dieser Methode im industriellen Maßstab sind und die es erlauben, die gewünschten optischen Antipoden der allgemeinen Formel (I) getrennt kostengünstiger zur Verfügung zu stellen.

Besonders ist ein Verfahren bevorzugt, das das Racemat des (±)-Azabicyclo(2.2.1)-hept-5-en-3-on in die Enantiomere erfindungsgemäß auftrennt.

Die Trennung der betrachteten Enantiomere im technischen Maßstab kann im Prinzip nach den für den Fachmann für diesen Zweck in Frage kommenden LC-Methoden erfolgen. So eignen sich die Trennungen mit sogenannten "pancake-Säulen" in der diskontinuierlichen Chromatographiemethode. Besonders bevorzugt ist jedoch die Anwendung der quasikontinuierlichen zyklischen Flüssigkeitschromatographie ggf. mit "recycling" und "peak-shaving" (J. Chromatogr. 1994, 666, 627-650) oder der kontinuierlichen SMB-Chromatographiemethode (Mazzotti et al. Chiral Europe 1996, 103f.; Strube et al. Organic Process Research & Development 1998, 2, 305-319; Juza et al. GIT Spezial Chromatographie 1998, 2, 108f.; EP0878222; Schulte et al. Chemie Ingenieur, Technik 1966, 68, 670-683).

Als Zuckerderivate kommen vorzugsweise solche auf Basis von Amylose oder Cellulose in Frage. Verschiedene derart modifizierte Derivate sind im Stand der Technik bekannt (Bull. Chem. Soc. Jpn. 1995, 68, 3209-3307). Geeignet sind vor allem Ester und Carbamate dieser Materialien.

Die Zuckerderivate können dabei nativ oder auf ein Trägermaterial aufgezogen eingesetzt werden. Als Trägermaterial eignet sich bevorzugt Silicagel. Ganz besonders vorteilhaft wird ein Material benutzt, welches auf Silicagel aufgezogene Zuckerderivate oder mikrokristalline Celluloseester aufweist. Äußerst bevorzugt ist der Einsatz von amylosederivatisierten Silicagelsäulen als stationäre Phase (z.B. Chiralpak AS® der Firma Daicel).

Das eingesetzte Laufmittel sollte Acetonitril aufweisen. Zur Verbesserung der Trenneigenschaften kann der Fachmann jedoch im Rahmen seines Fachwissens andere Lösungsmittel, welche für die HPLC geeignet erscheinen, dem Acetonitril zumischen. Bevorzugt ist jedoch die Variante bei der Acetonitril die Hauptkomponente am Laufmittelgemisch ausmacht. Als bevorzugt zuzumischendes Lösungsmittel hat sich Methanol erwiesen. Ganz besonders bevorzugt ist die Ausführungsform, bei der Acetonitril und Methanol als Hauptkomponenten im Laufmittelgemisch vorkommen.

Der Druck oder die Temperatur, welche für die

Trennung gewählt werden sollte, kann der Fachmann im Prinzip frei wählen. Die Parameter sollten so aufeinander abgestimmt sein, daß eine optimale Trennleistung bei möglichst hoher Durchsatzrate erzielt wird. Bevorzugt liegt der anzuwendende Druckbereich bei 0,3-2,0 MPa, vorzugsweise 0,5-0,7 MPa, die Temperatur bei 20-40°C, vorzugsweise 22-28°C.

Die vorliegende erfindungsgemäße Trennmethode erfolgt in einer gegenüber enzymatischen Verfahren sehr robusten Art und Weise. Die eingesetzten Materialien haben lange Standzeiten und die Methode erlaubt auch die kontinuierliche Herstellung der Enantiomere, was gerade für einen technischen Prozeß äußerst vorteilhaft ist.

Mithin wurde eine chromatographische Enantiomerentrennung dieser Substanzen im Stand der Technik noch nie erwähnt. Die Auswahl im Hinblick auf Säulenmaterial und Laufmittel aus den abertausenden von Kombinationsmöglichkeiten macht die einfache und doch so erfolgreiche Trennung der Enantiomere möglich.

Unter Schutzgruppe versteht man im Rahmen der Erfindung ein Rest ausgewählt aus der Gruppe: Formyl, Acetyl, Propionyl, Methoxycarbonyl, Ethoxycarbonyl, tert.-Butoxycarbonyl, Z, Fmoc, Benzyl.

Die gestrichelte Linie in der Formel (I) bedeutet, daß hier optional eine Doppelbindung vorhanden sein kann.

Als Flüssigkeitschromatographie wird im Sinne der Erfindung die Low Pressure Liquid Chromatography (LPLC), Medium Pressure Liquid Chromatography (MPLC) oder High Pressure Liquid Chromatography (HPLC) verstanden.

Der Trennfaktor wird wie folgt berechnet:

$$\text{Trennfaktor} = (T_o - T_1)/(T_o - T_2)$$

$T_o$ = 1,3,5-Tert.-butylbenzol

Beispiel:

HPLC-Trennung von (±)-Azabicyclo(2.2.1)-hept-5-en-3-on an chiralen Säulen

Probe: Jeweils 10 mg (±)-Azabicyclo(2.2.1)-hept-5-en-3-on sowie 1,3,5-Tri-tert.-butylbenzol (Fluka) als $T_0$-Marker werden in einen 10 ml Meßkolben eingewogen und mit dem jeweiligen Eluenten bis zur Merke aufgefüllt. 20µl dieser Lösung werden injiziert.

a)

Säule: Chiralpak® AS 250x4,6 mm (Daicel)
Eluent: Acetonitril
Fluß: 0,6 ml/min
Temperatur: 25°C
Wellenlänge: 230 nm

Ergebnis siehe Fig. 1
Trennfaktor α = 1,6

b)

Säule: Chiralpak® AS 250x4,6 mm (Daicel)
Eluent: Acetonitril/Methanol 80:20 (v/v)
Fluß: 0,6 ml/min
Temperatur: 25°C
Wellenlänge: 230 nm

Ergebnis siehe Fig. 2
Trennfaktor α = 1,6

c)

Säule: Chiralpak® AS 250x4,6 mm (Daicel)
Eluent: Acetonitril/Ethanol 80:20 (v/v)
Fluß: 0,6 ml/min
Temperatur: 25°C
Wellenlänge: 230 nm

Ergebnis siehe Fig. 3
Trennfaktor α = 1,51

d)

Säule: Chiralpak® AS 250x4,6 mm (Daicel)
Eluent: Acetonitril/2-Propanol 80:20 (v/v)
Fluß: 0,6 ml/min
Temperatur: 25°C
Wellenlänge: 230 nm

Ergebnis siehe Fig. 4
Trennfaktor α = 1,42

e)

Säule: Chiralpak® AD 250x4,6 mm (Daicel)
Eluent: Acetonitril
Fluß: 0,6 ml/min
Temperatur: 25°C
Wellenlänge: 230 nm

Ergebnis siehe Fig. 5
Trennfaktor 1,17

Quasikontinuierliche zyklische Varainte

Säule: Chiralpak® AD 250x40 mm (Daicel)
Säulenfüllung: 220 g des Phasenmaterials (20µm) in 1400 ml Acetonitril suspendieren und in das Füllrohr überführen, mit Acetonitril bis zum oberen Rand überschichten und das Füllrohr fest verschließen, bei 150 bar das Säulenmaterial verdichten
Eluent: Acetonitril

Fluß: 60 ml/min
Druck: ca. 60-80 bar
Temperatur: 22°C
Wellenlänge: 230 nm

Probenvorbereitung: 150 mg (±)-Azabicyclo (2.2.1)-hept-5-en-3-on werden in 15 ml Eluent gelöst und über einen Membranfilter filtriert. Die Gesamtmenge des klaren Filtrats wird auf die Säule gegeben. Etwa 1,5 g des Racemats wurde insgesamt chromatographiert.

Fraktionierung: Die Trennung des Racemats erfolgt in sechs Zyklen. Zyklus 1 wird komplett recycliert. Ab Zyklus 2 erfolgt die Fraktionierung der einzelnen Antipoden an den im Chromatogramm (Fig. 6) markierten Punkten.

Aufarbeitung: Die vereinigten Fraktionen der einzelnen Zyklen werden am Rotationsverdampfer bei ca. 250-350 mbar und einer Wasserbadtemperatur von etwa 40-45°C bis zur Trockene eingeengt.

Ergebnis: In 85%iger Ausbeute erhielt man die (+)-Antipode mit einem ee-Wert von >97,5% und die (-) -Antipode mit einem ee-Wert von 98%.

### Patentansprüche

1. Verfahren zur Enantiomerentrennung eines Isomerengemisches eines Lactams der allgemeinen Formel (I)

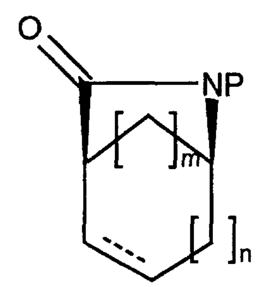

(I)

worin n, m unabhängig voneinander die Werte 0 bis 3 annehmen können,
P = H oder eine N-Schutzgruppe ausgewählt aus der Gruppe: Formyl, Acetyl, Propionyl, Methoxycarbonyl, Ethoxycarbonyl, tert.-Butoxycarbonyl, Z, Fmoc, Benzyl bedeutet,
durch Flüssigkeitschromatographie an Zuckerderivate aufweisenden chiralen Phasen mittels eines Eluenten, der Acetonitril aufweist.

2. Verfahren nach Anspruch 1, **dadurch gekennzeichnet, daß** man als Lactam (±)-Azabicyclo(2.2.1)-hept-5-en-3-on einsetzt.

3. Verfahren nach Anspruch 1 und/oder 2, **dadurch gekennzeichnet, daß** man die zyklische oder SMB-Chromatographiemethode anwendet.

4. Verfahren nach einem oder mehreren der vorhergehenden Ansprüche, **dadurch gekennzeichnet, daß** man als Zuckerderivate Ester oder Carbamate der Amylose oder Cellulose einsetzt.

5. Verfahren nach einem oder mehreren der vorhergehenden Ansprüche, **dadurch gekennzeichnet, daß** man als stätionäre Phase ein Material benutzt, welches auf Silicagel aufgezogene Zuckerderivate oder mikrokristalline Celluloseester aufweist.

6. Verfahren nach einem oder mehreren der vorhergehenden Ansprüche, **dadurch gekennzeichnet, daß** man als stationäre Phase eine amylosederivatisierte Silicagelsäule verwendet.

7. Verfahren nach einem oder mehreren der vorhergehenden Ansprüche, **dadurch gekennzeichnet, daß** das eingesetzte Laufmittel Acetonitril oder Acetonitril und Methanol als Hauptkomponenten aufweist.

8. Verfahren nach einem oder mehreren der vorhergehenden Ansprüche, **dadurch gekennzeichnet, daß** man bei einem Druck von 0,3-2,0 MPa, vorzugsweise 0,5-0,7 MPa, arbeitet.

9. Verfahren nach einem oder mehreren der vorhergehenden Ansprüche, **dadurch gekennzeichnet, daß** man bei einer Temperatur von 20-40°C, vorzugsweise 22-28°C, arbeitet.

### Claims

1. Process for the separation of enantiomers of a mixture of isomers of a lactam of the general formula (I)

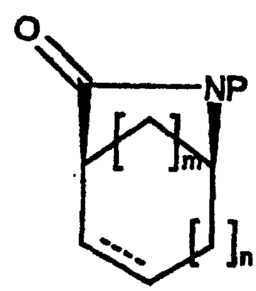

(I)

wherein n and m, independently of one another, can take on the values of 0 to 3,
P has the meaning of H or an N protective group selected from the group: formyl, acetyl, propionyl, methoxycarbonyl, ethoxycarbonyl, tert.-butoxycarbonyl, Z, Fmoc, benzyl,
by liquid chromatography on chiral phases containing sugar derivatives by means of an eluent containing acetonitrile.

**2.** Process according to claim 1,
**characterised in that**
(±)-azabicyclo(2.2.1)-hept-5-en-3-one is used as the lactam.

**3.** Process according to claim 1 and/or 2,
**characterised in that**
the cyclic or SMB chromatography method is used.

**4.** Process according to one or more of the above claims,
**characterised in that**
esters or carbamates of amylose or cellulose are used as the sugar derivatives.

**5.** Process according to one or more of the above claims,
**characterised in that**
a material containing sugar derivatives or microcrystalline cellulose esters coated on silica gel is used as the stationary phase.

**6.** Process according to one or more of the above claims,
**characterised in that**
an amylose-derivatised silica gel column is used as the stationary phase.

**7.** Process according to one or more of the above claims,
**characterised in that**
the mobile phase used contains acetonitrile or acetonitrile and methanol as the main components.

**8.** Process according to one or more of the above claims,
**characterised in that**
work is carried out under a pressure of 0.3 - 2.0 MPa, preferably 0.5 - 0.7 MPa.

**9.** Process according to one or more of the above claims,
**characterised in that**
work is carried out at a temperature of 20 - 40°C, preferably 22 - 28°C.

**Revendications**

**1.** Procédé pour la séparation d'énantiomères d'un mélange d'isomères d'un lactame de formule générale (I)

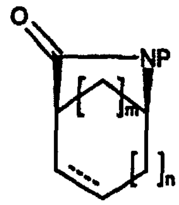

(I)

dans laquelle n, m peuvent adopter, indépendamment l'un de l'autre, les valeurs 0 à 3,
P = H ou représente un groupe protecteur d'azote choisi dans l'ensemble constitué par les groupes formyle, acétyle, propionyle, méthoxycarbonyle, éthoxycarbonyle, tert-butoxycarbonyle, Z, Fmoc, benzyle,
par chromatographie liquide sur des phases chirales comportant des dérivés glucidiques, au moyen d'un éluant qui comporte de l'acétonitrile.

**2.** Procédé selon la revendication 1,
**caractérisé en ce qu'**
on utilise comme lactame la (±)-azabicyclo(2.2.1)-hept-5-én-3-one.

**3.** Procédé selon la revendication 1 et/ou la revendication 2,
**caractérisé en ce qu'**
on utilise la méthode de chromatographie cyclique ou SMB.

**4.** Procédé selon une ou plusieurs des revendications précédentes,
**caractérisé en ce qu'**
on utilise comme dérivés de glucides des esters ou carbamates de l'amylose ou de la cellulose.

**5.** Procédé selon une ou plusieurs des revendications précédentes,
**caractérisé en ce qu'**
on utilise comme phase stationnaire un matériau qui comporte des esters de cellulose microcristallins ou des dérivés de glucides fixés sur gel de silice.

**6.** Procédé selon une ou plusieurs des revendications précédentes,
**caractérisé en ce qu'**
on utilise comme phase stationnaire une colonne de gel de silice transformé en dérivé avec de l'amylose.

**7.** Procédé selon une ou plusieurs des revendications

précédentes,
**caractérisé en ce que**
l'éluant utilisé comporte comme composants principaux de l'acétonitrile ou de l'acétonitrile et du méthanol.

**8.** Procédé selon une ou plusieurs des revendications précédentes,
**caractérisé en ce qu'**
on opère sous une pression de 0,3 à 2,0 MPa, de préférence de 0,5 à 0,7 MPa.

**9.** Procédé selon une ou plusieurs des revendications précédentes,
**caractérisé en ce qu'**
on opère à une température de 20-40°C, de préférence de 22-28°C.

Fig. 1

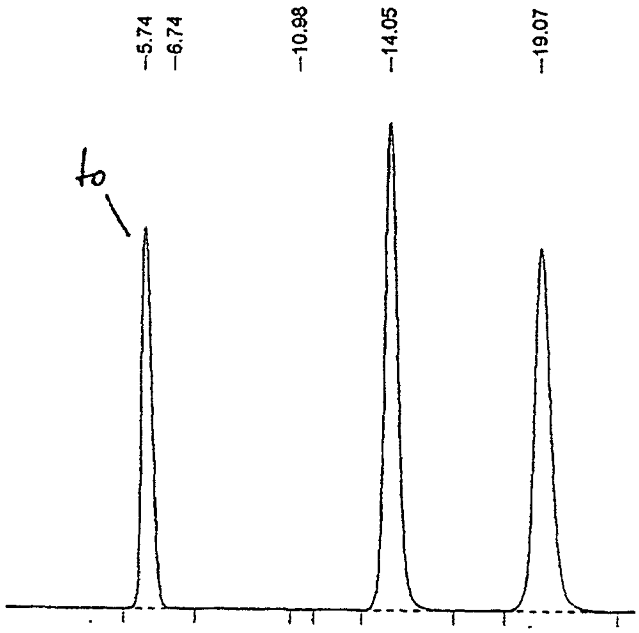


Fig.2

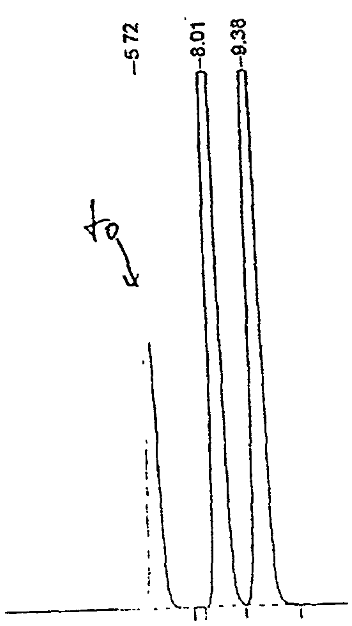

Fig. 3

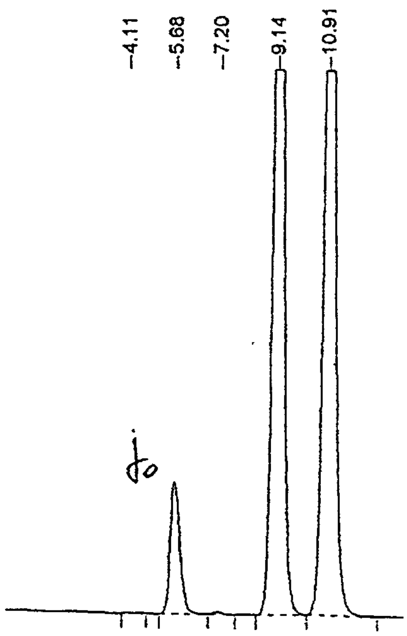


Fig. 4

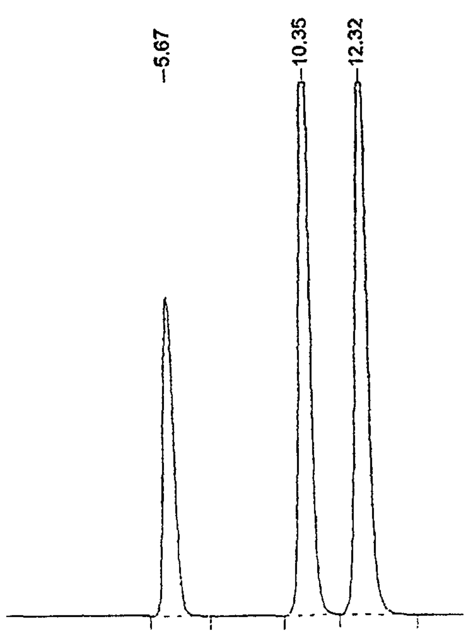

Fig. 5

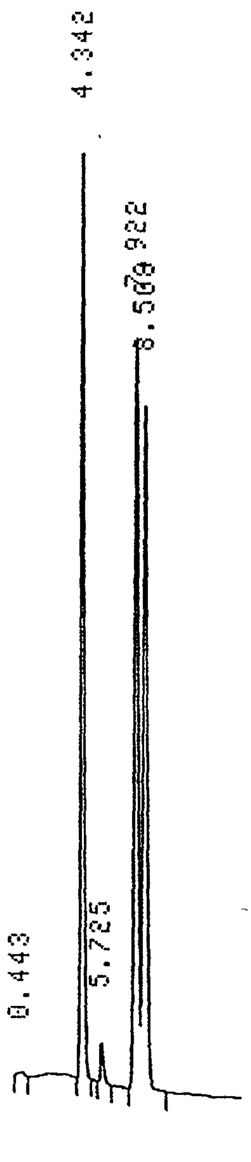

Fig. 6

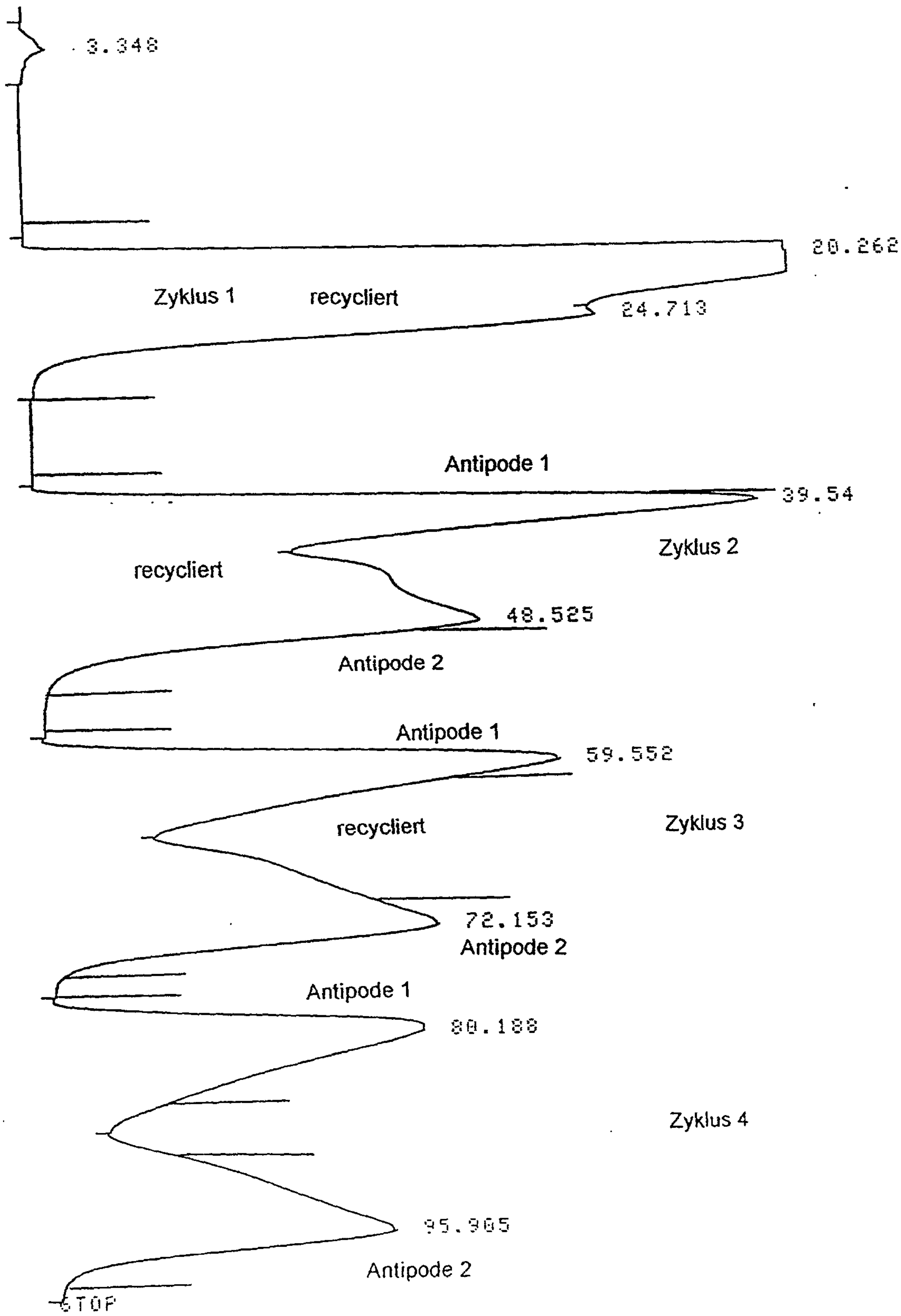
3.348
20.262
Zyklus 1
recycliert
24.713
Antipode 1
39.54
Zyklus 2
recycliert
48.525
Antipode 2
Antipode 1
59.552
recycliert
Zyklus 3
72.153
Antipode 2
Antipode 1
80.188
Zyklus 4
95.905
Antipode 2
STOP